# FASCICULE DE BREVET EUROPEEN

(11) **EP 4 347 012 B1**
(45) Date de publication et mention de la délivrance du brevet: **06.05.2026**
(21) Numéro de dépôt: 22731743.5
(22) Date de dépôt: 20.05.2022
(51) Int. Cl.: A61N 5/06

(54) **DISPOSITIF DE TRAITEMENT DE PHOTOTHÉRAPIE**
VORRICHTUNG ZUR PHOTOTHERAPIEBEHANDLUNG
PHOTOTHERAPY TREATMENT DEVICE

(30) Priorité: 27.05.2021 FR 2105535
(43) Date de publication de la demande: 10.04.2024
(73) Titulaire: Lucibel SA, 76360 Barentin (FR)
(72) Inventeur: GROS DAILLON, Thibault, 76590 CRIQUETOT-SUR-LONGUEVILLE (FR); DAVID, Thibault, 94410 SAINT-MAURICE (FR)
(74) Mandataire: Martin, Marie-Aude
(86) Numéro de dépôt international: PCT/FR2022/050960
(87) Numéro de publication internationale: WO 2022/248793

(56) Documents cités:
- CN-A- 108 465 159
- CN-A- 108 465 159
- FR-A1- 3 034 022
- FR-A1- 3 034 022
- KR-A- 20110 117 481
- KR-A- 20110 117 481
- KR-B1- 101 418 294
- KR-B1- 101 418 294
- US-A1- 2018 352 936
- US-A1- 2018 352 936

## Description

La présente invention concerne un dispositif destiné à fournir un traitement de photothérapie ou photo-modulation pour améliorer la santé de la peau, tel qu'un traitement anti-âge ou un traitement de prévention de l'acné, en utilisant la photothérapie par diode électroluminescente (DEL), bien que d'autres types de sources de rayonnement lumineux puissent être utilisées.

Certains spectres lumineux émis par les DELs (bleus ou rouges) sont connus pour être efficaces pour le traitement des affections de la peau telles que l'acné ou pour inhiber le vieillissement cutané.

Il est ainsi souhaitable de fournir aux utilisateurs un dispositif pratique de photothérapie à domicile, tel qu'un masque, qui soit simple à utiliser sans gêne pour l'utilisateur. Un tel masque est notamment connu du document US2018/0352936.

Les produits actuellement disponibles à domicile et utilisables par le consommateur sur le marché sont fixés à une taille unique et/ou doivent généralement être tenus à la main, ce qui ne s'est généralement pas avéré satisfaisant pour assurer la meilleure diffusion de la lumière. L'alternative est que les clients se rendent dans un institut spécialisé pour recevoir des traitements.

Les appareils de photothérapie connus dans l'art antérieur, en particulier les masques, présentent de nombreux problèmes liés à l'exposition des DELs et des circuits associés pour alimenter les DELs au contact des utilisateurs.

Plus particulièrement, dans le but de maximiser le flux lumineux sur le visage de l'utilisateur, les DELs sont généralement disposées de manière à permettre à ce dernier d'être en contact physique avec les DELs, ce qui provoque l'accumulation de poussière et d'huile à proximité ou sur les DELs. De plus, un tel contact peut être dangereux pour les utilisateurs qui sont exposés aux bords tranchants ou chauds des DELs et des circuits associés.

L'exposition directe aux circuits et aux DELs peut constituer en outre une expérience intimidante et désagréable lorsque le traitement prend plusieurs minutes et que le masque est disposé relativement près du visage, provoquant souvent une sensation de claustrophobie et d'inconfort chez l'utilisateur au cours de la durée de traitement.

Il a été observé chez certains utilisateurs, que certaines régions du visage sont susceptibles de rapidement rougir même lors d'une durée d'exposition médicalement approuvée. Ceci se traduit notamment par des rougeurs persistantes dans certaines parties du visage plus sensibles tel que le contour des yeux.

Pour y remédier, l'utilisateur est contraint soit de limiter la durée d'exposition au traitement de photothérapie soit d'utiliser des dispositifs de photothérapie ayant une surface d'émission plus petite qui permet un traitement plus ciblé et localisé du visage.

Il existe donc un besoin d'un masque facial qui réponde à la fois au besoin de traiter de façon efficace l'ensemble du visage tout en limitant les risques de surexposition à la lumière rouge.

A cet effet, l'invention a pour objet un masque facial de traitement par photothérapie d'un visage d'un utilisateur, comprenant une armature délimitant une face frontale orientée en direction d'un visage d'un utilisateur et une face dorsale opposée, l'armature supportant une pluralité de points lumineux d'émission lumineuse agencés dans le masque pour produire un éclairage énergétique du visage de l'utilisateur par la face frontale, caractérisé en ce que le masque facial définissant, en correspondance faciale, au moins une ligne remarquable dite méridienne du front au menton et une pluralité de lignes remarquables dites parallèles s'étendant entre chaque côté du visage sensiblement transversalement à la ligne méridienne, les points lumineux sont disposés essentiellement le long des lignes parallèles afin de produire une distribution spatiale de l'éclairement énergétique du visage présentant une densité de puissance lumineuse maximale le long de ces lignes et en ce que la densité de puissance lumineuse générée par les points lumineux varie d'une ligne à l'autre ou le long d'une même ligne afin de définir des régions cibles de forte puissance lumineuse et des régions cibles de plus faible puissance lumineuse le long de la ligne ou d'une ligne à l'autre, dans lequel le taux de dosage énergétique lumineux variant le long d'une même ligne et/ou d'une ligne à l'autre permet d'ajuster le taux de dosage énergétique lumineux dans une ligne orbitale inférieure et une ligne orbitale supérieure définissant une région péri-orbitale de telle sorte que l'éclairement énergétique dans la région péri-orbitale est moins élevé que dans une région malaire ou maxillaire du visage.

Grâce au masque de l'invention, la distribution des sources de lumières ou points lumineux permet définir des régions cibles de forte puissance lumineuse et des régions cibles de plus faible puissance lumineuse le long de la ligne ou d'une ligne à l'autre. Cette distribution permet de garantir une efficacité thérapeutique optimale du traitement du visage humain.

Un dispositif de traitement selon l'invention peut en outre comporter l'une ou plusieurs des caractéristiques suivantes.

Selon un mode de réalisation préféré de l'invention, les points lumineux présentent une distribution spatiale observable depuis la face frontale du masque définissant un motif de réseau de lignes distinctes et discernables les unes des autres.

Selon un mode de réalisation préféré de l'invention, le visage pouvant être décomposé en une pluralité de zones susceptibles d'être marquées par les rides, le réseau définit une pluralité de lignes de points lumineux agencées pour traverser lesdites zones, la longueur de chaque ligne du réseau étant dimensionnée en fonction d'une étendue transversale de la zone traversée par la ligne.

Selon un mode de réalisation préféré de l'invention, le réseau comprend au moins une ligne curviligne, par exemple pour suivre un contour péri-orbital ou péri-buccal du visage.

Selon un mode de réalisation préféré de l'invention, la densité de puissance lumineuse générée par les points lumineux varie le long d'une même ligne et/ou d'une ligne à l'autre.

Selon un mode de réalisation préféré de l'invention, la densité de puissance lumineuse maximale (éclairement énergétique connu également selon la terminologie anglo-saxonne par « irradiance ») générée par les points lumineux pour une ligne remarquable a une valeur comprise entre 5 et 100 mW/cm², en particulier comprise entre 20 et 70 mW/cm².

Selon un mode de réalisation préféré de l'invention, la densité linéique des points lumineux varie le long d'une ligne et/ou d'une ligne à l'autre.

Selon un mode de réalisation préféré de l'invention, définissant une ligne circonférentielle qui suit une forme générale ovale périphérique du visage le long de laquelle est agencée une pluralité de points lumineux.

Selon un mode de réalisation préféré de l'invention, la ligne parallèle est choisie parmi une ligne orbitale inférieure, une ligne orbitale supérieure, une ligne maxillaire inférieure, une ligne frontale, une ligne maxillaire supérieure, une ligne malaire centrale.

Selon un mode de réalisation préféré de l'invention, chaque point lumineux étant formé par une diode électroluminescente, l'armature comprend un support de raccordement électrique des diodes électroluminescentes entre elles, ledit support comprenant au moins un tronc principal s'étendant sensiblement selon la ligne méridienne et au moins deux branches s'étendant sensiblement selon au moins deux lignes parallèles distinctes.

Selon un mode de réalisation préféré de l'invention, chaque branche s'étend symétriquement par rapport à un plan contenant la ligne méridienne.

Selon un mode de réalisation préféré de l'invention, comprenant une pluralité de branches primaires reliées directement au tronc et au moins une branche secondaire reliée indirectement au tronc par l'intermédiaire d'au moins une branche primaire.

Selon un mode de réalisation préféré de l'invention, le tronc principal comprend un bornier de raccordement à une extrémité d'un câble d'alimentation électrique des sources lumineuses.

Selon un mode de réalisation préféré de l'invention, l'armature comprend deux parois en matière plastique conformée pour présenter une concavité orientée en direction du visage à traiter et entre lesquelles s'étend le support portant les points lumineux.

Selon un mode de réalisation préféré de l'invention, l'armature comprenant une plaque de dissipation thermique conformée pour venir épouser la concavité du corps et disposée entre les deux parois, sur lequel est monté le support du côté de la plaque faisant face à la paroi intérieure.

Selon un mode de réalisation préféré de l'invention, l'armature est textile et comprend une pluralité de fibres optiques en chaîne et/ou en trames tissées avec des fils de liage des fibres de l'armature, chaque fibre optique comprenant un corps longitudinal pourvu selon sa direction longitudinale d'altérations invasives ouvertes sur la face frontale de l'armature et d'au moins une extrémité libre en regard de laquelle est agencée une source d'émission de lumière pour transmettre et émettre la lumière au niveau des altérations de la face frontale pour former la pluralité de points lumineux.

Selon un mode de réalisation préféré de l'invention, la densité linéique et/ou la dimension des altérations varie le long de la fibre optique ou d'une fibre optique à l'autre.

Selon un mode de réalisation préféré de l'invention, l'armature en toile et ou en satin et dans lequel les fils de liage sont formés dans un matériau choisi parmi le groupe comprenant le polyamide le polyester le polyéthylène et le polypropylène.

Selon un mode de réalisation préféré de l'invention, les points lumineux émettent dans un spectre rouge de longueur d'onde comprise entre 620 et 640 nm.

Les dessins annexés illustrent l'invention :
[Fig.1] représente une vue en perspective d'un dispositif de photothérapie selon un premier mode de réalisation de l'invention ;
[Fig.2] représente une vue en perspective d'un visage humain dans lequel ont été identifiées un premier ensemble de lignes remarquables ;
[Fig.3] représente une vue partielle en perspective éclatée du dispositif de la figure 1 ;
[Fig.4] représente une vue d'une face frontale du masque facial du dispositif de la figure 1 dans lequel une paroi intérieure est absente ;
[Fig.5] représente une vue de face d'un support de raccordement électrique d'une pluralité de points lumineux du masque de la figure 4 ;
[Fig.6] représente une vue en perspective du support de raccordement électrique de la figure 4 monté sur un socle du dispositif de la figure 1 ;
[Fig.7] représente une vue en perspective d'une plaque de dissipation thermique montée à l'intérieur du masque du dispositif de la figure 1 ;
[Fig.8] représente une vue en perspective d'une variante du support de raccordement électrique du masque du dispositif de la figure 1 ;
[Fig.9] représente une vue en perspective d'une variante du support de raccordement électrique du masque du dispositif de la figure 1 ;
[Fig.10] représente une vue en perspective d'une variante du support de raccordement électrique du masque du dispositif de la figure 1 ;
[Fig.11] représente une vue en perspective d'un dispositif de photothérapie selon un deuxième mode de réalisation de l'invention ;

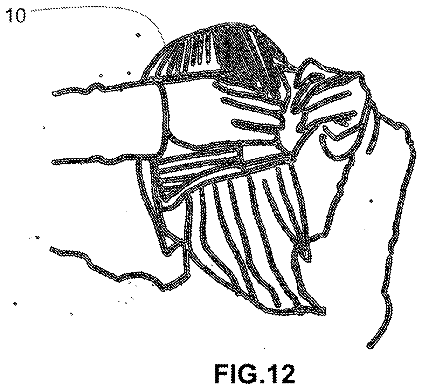

[Fig.12] représente une vue en perspective de la mise en place du dispositif de photothérapie selon le deuxième mode de réalisation de l'invention.

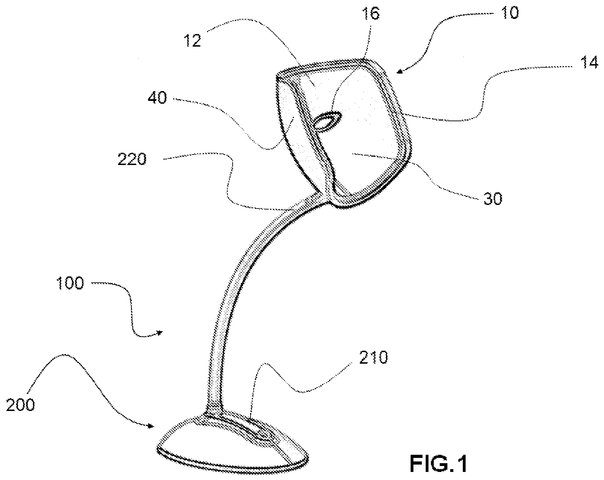

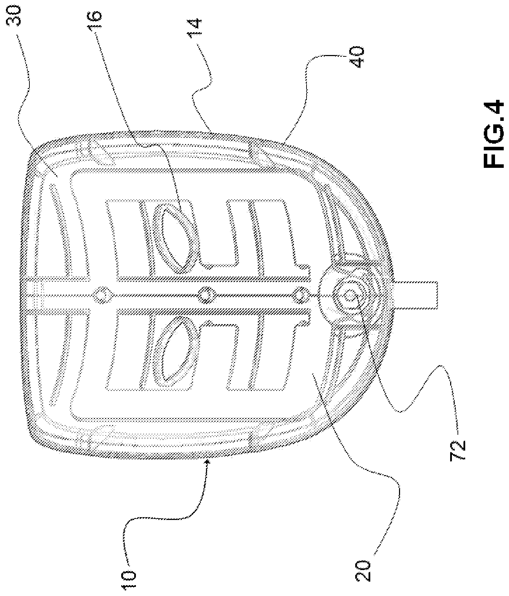

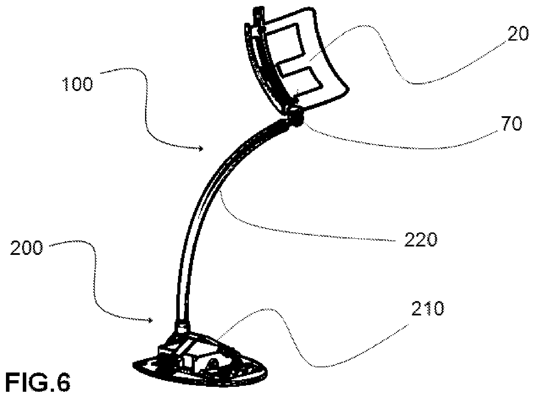

On a représenté sur les figures 1 à 10 un dispositif de traitement par photothérapie selon un premier mode de réalisation de l'invention. Ce dispositif est désigné par la référence générale 100.

Dans le premier mode de réalisation, le dispositif 100 comprend un masque facial 10 et un support 200 pour maintenir le dispositif de traitement 10 comme cela sera détaillé ci-après.

Comme cela est illustré en détail sur la figure 1, le masque facial 10 comprend une armature 12 relativement rigide de forme générale concave délimitée par un bord périphérique 14, destinée à être positionnée de préférence en regard d'une zone à traiter d'un utilisateur, ici le visage de l'utilisateur. Dans l'exemple décrit, l'armature 12 est formée d'une coque rigide conformée pour venir recouvrir l'ensemble du visage du patient dans une position d'utilisation.

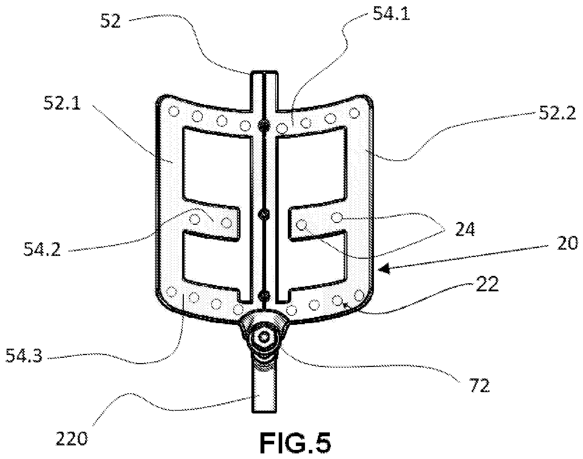

En outre, l'armature 12 comprend encore une pluralité de points lumineux 22 d'émission lumineuse, illustrés en détail sur la figure 5.

Dans ce premier mode de réalisation, les points lumineux 22 comprennent au moins chacune une diode électroluminescente 24 avec un spectre de couleur rouge dans une longueur d'onde comprise principalement et de préférence entre 620 et 640 nm. De préférence, le flux lumineux émis par chaque diode électroluminescente 24 est au minimum de 30 lumens, par exemple compris dans la plage 30 lumens - 60 lumens.

Dans l'exemple illustré sur la figure 1, l'armature 12 est conformée pour couvrir le visage en épousant la courbure naturelle du visage et présente ainsi une concavité orientée en direction de la face de l'utilisateur. Cette concavité permet de recouvrir le visage d'un être humain en suivant la courbure naturelle entre les zones malaires d'une part et entre la zone frontale et la zone maxillaire inférieure d'autre part.

Dans cet exemple, la concavité du masque 10 permet notamment de mieux épouser et suivre les contours du visage d'un utilisateur et ainsi rendre plus efficace l'exposition au rayonnement lumineux émis par les points lumineux 22.

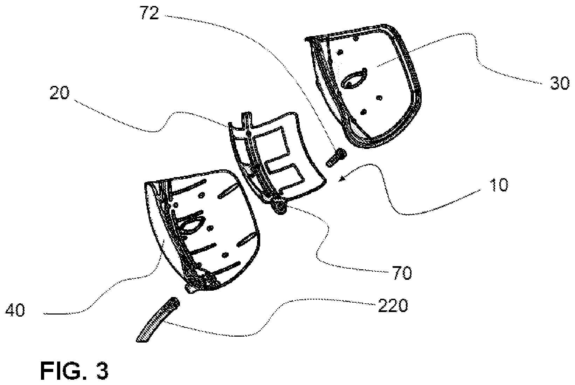

De préférence, comme cela est illustré sur la figure 3, l'armature 12 comprend une première paroi frontale intérieure 30 destinée à être positionnée en regard du visage de l'utilisateur. Cette paroi intérieure 30 est préformée et a une forme générale concave, par exemple curviligne pour s'adapter à la physionomie d'un visage humain. Ainsi, de préférence, comme cela est illustré en figure 3, la paroi intérieure 30 a une forme générale de masque facial et comprend une région centrale présentant au moins une courbure pour suivre la courbure naturelle d'un visage d'être humain.

La première paroi intérieure 30 est réalisée dans une matière laissant passer le flux lumineux des diodes électroluminescentes 24. De préférence, la matière est translucide pour permettre une diffusion homogène de la lumière ainsi qu'une protection naturelle de l'utilisateur contre l'éblouissement. Par translucide, on entend un matériau « qui laisse passer la lumière mais qui n'est pas transparent ». Le choix d'un matériau translucide plutôt qu'un matériau transparent permet également au niveau esthétique de rendre moins visibles les sources lumineuses 24. Bien entendu, en variante, le matériau peut être choisi transparent aux rayons lumineux émis par les sources lumineuses 24.

En outre, de préférence, la paroi intérieure 30 est réalisée dans une matière plastique thermoformable, par exemple un mélange de polystyrène et de polyéthylène (acronyme PS-PE).

La paroi intérieure 30 est ainsi de préférence formée à partir d'un matériau susceptible d'évoluer en fonction de la température entre une phase plastique et une phase rigide, par modelage dudit matériau dans sa phase plastique dans un moule, puis rigidification dudit matériau en place dans le moule.

En outre, conformément à l'invention, le dispositif 10 comprend une deuxième paroi 40 conformée pour venir épouser, dans sa configuration finale, la première paroi 30.

Conformément à l'invention, les sources lumineuses 22 sont intercalées entre les deux parois intérieure 30 et extérieure 40.

Le procédé de fabrication générale de l'armature 12 comprend par exemple les étapes successives de (a) découpe d'une pièce dudit matériau, aux dimensions du moule selon une forme s'étalant dans le moule, et (b) mise en forme de ladite pièce, par modelage de la pièce de matériau dans sa phase plastique, (c) rigidification du matériau modelé, en place dans le moule pour former les parois de la coque rigide (12), et (d) aménagement d'évidements au niveau oculaire, soit sur la pièce avant sa mise en forme, soit une fois les parois de la coque rigidifiée.

De préférence, les parois 30 et 40 sont accouplées entre elles par des attaches, par exemple en matière plastique se présentant sous forme de clips (non représentés), et traversant des orifices coïncidant des deux parois 30 et 40. A cet effet, la paroi interne 30 présente une pluralité d'orifices et la paroi externe 40 comprend une pluralité d'orifices de fixation correspondants.

En outre, comme cela est illustré sur la figure 3, dans le premier mode de réalisation de l'invention, le masque 10 comprend un support 20 de raccordement électrique des sources lumineuses 24 à une source d'alimentation électrique. Dans ce premier mode de réalisation, le support 20 a une forme générale de plaque ajourée suffisamment déformable pour épouser la concavité du masque 10.

Selon l'invention, on considère pour le masque facial 10, en correspondance faciale, au moins une ligne remarquable dite méridienne M1 du front au menton et au moins une ligne remarquable dite parallèle P prédéfinie s'étendant entre chaque côté du visage.

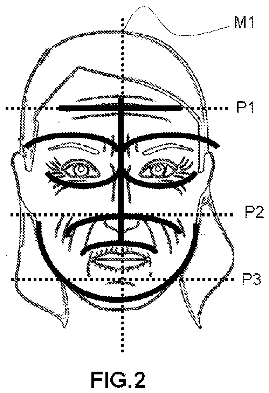

On considérera dans la suite de la description une décomposition d'un visage humain selon des lignes parallèles P prédéfinies remarquables pour le traitement des rides s'étendant transversalement à une ligne méridienne principale M1 qui s'étend du front jusqu'à la région maxillaire supérieure, telle que cela est illustré schématiquement par la figure 2 :
- une première ligne parallèle frontale P1 remarquable par exemple pour le traitement des rides du front ;
- une deuxième ligne parallèle malaire principale P2 s'étendant d'un côté à l'autre du visage pour le traitement par exemple des rides du sillon nasogénien ,
- une troisième ligne parallèle P3 maxillaire s'étendant au niveau de la bouche de l'utilisateur pour le traitement par exemple des rides péri-buccales.

Selon l'invention, les points lumineux 22 sont agencés principalement le long de ces lignes parallèles (éventuellement de façon optionnelle le long de la ligne méridienne principale) afin de produire une distribution spatiale de l'éclairement énergétique du visage présentant une densité de puissance surfacique lumineuse maximale dans des régions localisées le long de ces lignes et minimale à distance de ces lignes. On comprendra par « principalement » que de préférence plus des trois quarts (voir plus de 90%) des points lumineux 22 sont agencés le long des lignes parallèles.

La ligne parallèle est par exemple choisie parmi une ligne orbitale inférieure, une ligne orbitale supérieure, une ligne maxillaire inférieure, une ligne frontale, une ligne maxillaire supérieure, une ligne malaire centrale. Les lignes parallèles particulières comprennent notamment une ligne frontale, une ligne maxillaire inférieure, une ligne maxillaire supérieure, une ligne malaire centrale et une ligne orbitale inférieure et une ligne orbitale supérieure.

De préférence, les points lumineux 22 présentent une distribution spatiale observable depuis la face frontale du masque 10 définissant un motif de réseau de lignes distinctes et discernables les unes des autres. Cette distribution en réseau est non uniforme sur le visage et au contraire se présente sous forme de lignes de réseau qui traversent des zones cibles du visage de l'utilisateur.

Dans l'exemple décrit, le visage pouvant être décomposé en une pluralité de zones susceptibles d'être marquées par les rides, le réseau définit une pluralité de lignes de points lumineux agencées pour traverser lesdites zones, la longueur de chaque ligne du réseau étant dimensionnée en fonction d'une étendue transversale de la zone traversée par la ligne.

Par exemple, la zone susceptible d'être marquée par les rides est choisie parmi une zone malaire comprenant les rides des joues et des sillons nasogéniens et mélolabiaux, une zone frontale comprenant les rides du front et les rides intersourcilières, une zone péri-orbitale comprenant les rides péri-orbitales et/ou une zone péri-buccale comprenant les rides autour de la bouche.

Par exemple, le motif de réseau comprend au moins une ligne curviligne, par exemple pour suivre un contour péri-orbital ou péri-buccal du visage.

Plus particulièrement, dans ce premier mode de réalisation, le support 20 délimite au moins un tronc principal 52 s'étendant sensiblement selon la ligne méridienne M1 et une pluralité de branches 54 s'étendant sensiblement selon au moins deux lignes parallèles distinctes P1 à P3.

Dans l'exemple illustré, le support 20 délimite un tronc principal 52 méridien et une branche frontale 54.1 et une branche maxillaire inférieure 54.3. Le support 20 comporte en outre deux branches méridiennes latérales 52.1 et 52.2 s'étendant sensiblement parallèlement au tronc principal méridien 52 permettant de s'étendre latéralement selon le contour du visage. Le support 20 comporte encore de préférence une branche malaire 54.2.

Comme cela est illustré sur la figure 5, afin de permettre de fournir une flexibilité accrue au support 20, cette branche malaire 54.2 est raccordée de façon secondaire au tronc principal méridien 52 par l'intermédiaire par exemple des branches méridiennes latérales 52.1 et 52.2. Les branches 54 du support 20 forment dans l'exemple illustré un agencement en double « E » disposés en vis-à-vis l'un de l'autre de façon symétrique par rapport au tronc méridien 52 auquel ils sont raccordés.

De préférence, le support 20 comprend une pluralité de branches dites « primaires » 54.1 et 54.3 car reliées directement au tronc 52 et au moins une branche dite « secondaire » 54.2 reliée indirectement au tronc 52 par l'intermédiaire d'au moins une branche primaire 54.1 ou 54.3

La décomposition du masque en lignes de dosage énergétique élevée permet d'optimiser l'apport énergétique de lumière dans des zones très spécifiques et ciblées du visage de l'utilisateur et de limiter l'apport énergétique de lumière dans des zones ne nécessitant pas ou peu de traitement de photothérapie.

En outre, de préférence, le taux de dosage énergétique lumineux (autrement considéré la densité surfacique de puissance lumineuse) varie le long d'une même ligne et/ou d'une ligne à l'autre ce qui permet également de pouvoir traiter certaines zones localisées (par exemple la région péri-orbitale) avec un ajustement du taux de dosage énergétique lumineux.

Par exemple, la variation de la densité surfacique de puissance lumineuse est obtenue en faisant varier la densité linéique des points lumineux le long d'une ligne et/ou d'une ligne à l'autre.

Dans cet exemple, la densité de puissance lumineuse maximale, ou éclairement énergétique, générée par les points lumineux 22 pour une ligne remarquable a une valeur comprise entre 5 et 100 mW/cm², en particulier entre 20 et 70 mW/cm².

Par exemple, l'éclairement énergétique dans une région péri-orbitale est de préférence proche de 25 mW/cm² (avec une marge de 20%). Dans une région malaire et maxillaire du visage, cet éclairement énergétique est de préférence plus élevé par exemple plus proche de 70 mW/cm² (avec une marge de 20%). Dans une région frontale du visage, cet éclairement énergétique est de préférence médian, par exemple proche de 40mW/cm² (avec une marge de 20%).

De préférence, la densité de puissance lumineuse émise sur chaque ligne méridienne ou parallèle par les points lumineux 22 varie le long d'une branche 54, ou d'une branche 54 à l'autre, ou d'une branche 54 par rapport au tronc 52.

Par exemple, la distance séparant deux sources lumineuses 22 le long d'une branche 54 est variable pour faire varier la densité de puissance. De préférence, chaque branche 54 s'étend symétriquement par rapport à un plan contenant la ligne méridienne M1.

Conformément à l'invention, les sources lumineuses 22 sont intercalées entre les deux parois intérieure 30 et extérieure 40. Par ailleurs, de préférence, la plaque de dissipation thermique 60 s'étend entre la paroi extérieure 40 et le support 20.

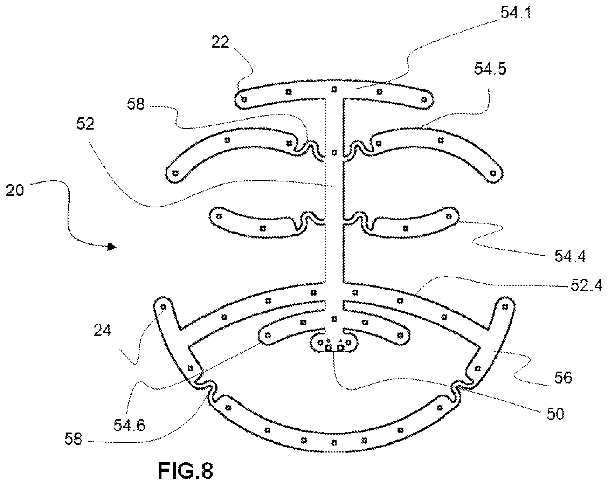

Dans la première variante illustrée sur la figure 8, le support 20 comprend toujours un tronc principal méridien 52 et une pluralité de branches parallèles 54 partant de ce tronc 52.

Les branches parallèles 54 ont dans cette variante des formes curvilignes pour suivre au plus près la courbure naturelle du visage. Dans cette variante, le support 20 délimite toujours une branche parallèle frontale 54.1, une branche malaire parallèle 54.3.

De façon générale, les lignes parallèles remarquables P du visage humain comprennent notamment une ligne frontale, une ligne maxillaire inférieure, une ligne maxillaire supérieure, une ligne malaire centrale et des lignes péri-orbitales (ligne orbitale inférieure et une ligne orbitale supérieure). On remarque sur la figure 2 que ces lignes (représentées en trait continu) sont curvilignes selon une direction transversale principale.

Ainsi, de préférence, dans cette variante, le support 20 délimite en outre des branches parallèles péri-orbitales inférieure 54.4 et supérieure 54.5 ainsi qu'une branche maxillaire supérieure 54.6. Par ailleurs, le support 20 comprend encore une branche semi-circonférentielle 56 secondaire reliée indirectement au tronc méridien par la branche 54.3, s'étendant sensiblement selon une ligne circonférentielle définie par une forme générale semi-ovale d'une partie inférieure du visage.

De préférence, dans cette variante, le support 20 comprend au moins un segment déformable 58 fournissant une flexion et une extension longitudinale du support 20 localisée sur le segment 58. La conformation en segment 58 élastiquement déformable fournit une flexion et une extension longitudinale du support 20 supplémentaires.

Dans la première variante, comme cela est illustré sur la figure 8, le segment déformable 58 peut avoir une forme générale de S ou une forme générale d'onde sinusoïdale. De façon générale, le segment 58 peut présenter une forme générale ondulée.

Par ailleurs, le support 20 comprend encore une branche semi-circonférentielle 56 secondaire reliée indirectement au tronc méridien par la branche 54.3, s'étendant sensiblement selon une ligne circonférentielle définie par une forme générale semi-ovale d'une partie inférieure du visage.

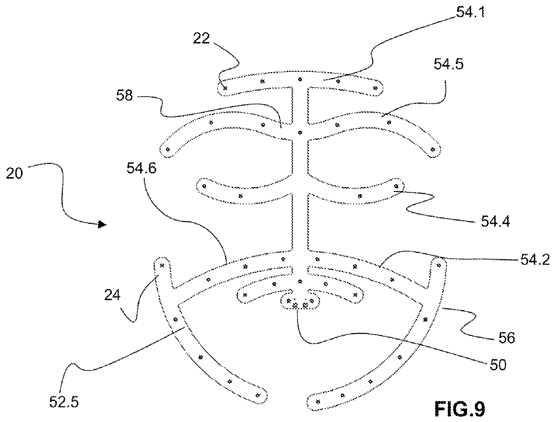

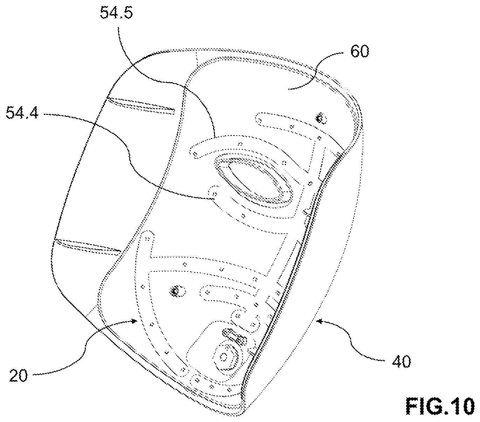

Dans la deuxième variante illustrée sur les figures 9 et 10, la branche demi-circonférentielle 56 secondaire est interrompue afin d'offrir une flexibilité améliorée du support 20.

Par ailleurs, comme cela apparaît très clairement sur la figure 10, les lignes de points lumineux 22 forment une distribution spatiale sous la forme d'un motif en réseau, visible depuis la face frontale du masque 10. Ce motif en réseau permet de suivre au mieux la cartographie de l'épiderme du visage par des lignes parallèles qui traversent les zones susceptibles d'être marquées par les rides.

Sur la figure 1, le support 200 comprend un socle 210 et un tube flexible 220 autoportant permettant de modifier également l'orientation du masque 10 de façon ergonomique. De préférence, le tube 220 flexible est configuré en « col de cygne » de façon à supporter le masque facial 10 tout en permettant de modifier également l'orientation du dispositif 10.

Un tube flexible dit « col de cygne » ou en anglais « stand tube » peut se déformer selon une trajectoire curviligne tout en assurant une fonction de support. De façon connue en soi, un tel tube est réalisé par exemple à partir d'une combinaison de deux enroulements généralement métalliques de sections ronde et triangulaire qui s'enroulent de façon alternée longitudinalement.

De préférence, le support 200 comprend une gaine en matière plastique qui recouvre le tube flexible 220 sur toute sa longueur afin de permettre la manipulation du tube flexible 220 dans des conditions d'hygiène optimales.

Le socle 210 forme dans cet exemple un boîtier enfermant une carte électronique de contrôle (non représentée) des sources lumineuses 22 selon un protocole de traitement prédéfini. En outre, le support 200 comprend un câble d'alimentation destiné à être connecté à une de ses extrémités à une source d'alimentation électrique, par exemple l'alimentation générale d'un réseau électrique domestique et à l'autre de ses extrémités au support de raccordement électrique des sources lumineuses 22. De préférence, le boîtier comprend également par exemple un convertisseur AC/DC classique. Ce câble d'alimentation électrique s'étend à l'intérieur du tube flexible.

A cet effet, de préférence, le support 20 comprend un bornier 50 de raccordement à une extrémité du câble d'alimentation électrique. Dans l'exemple illustré, le bornier 50 s'étend sur le tronc méridien 52, de préférence au voisinage d'une extrémité libre de ce tronc 52. De préférence, le bornier 50 de raccordement électrique comprend un agencement de type presse-étoupe 70 au niveau de l'extrémité du câble pour assurer une étanchéité et une stabilité mécanique du circuit de sources lumineuses 22 vis-à-vis de l'environnement extérieur. De façon connue en soi, un agencement de type presse-étoupe 70 comprend une garniture compressible, à l'origine de l'étoupe qui est comprimée à l'aide d'un écrou ou d'une vis annulaire 72. Un tel presse-étoupe 70 permet de maintenir et d'assurer l'étanchéité autour du câble électrique.

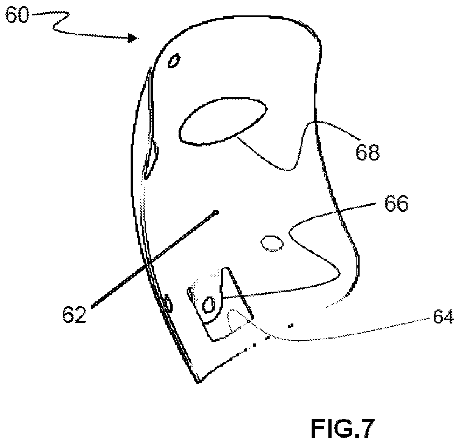

En outre, comme cela est illustré sur la figure 7, le masque facial 10 comprend encore une interface de dissipation thermique 60 réalisée par exemple en aluminium. Cette interface de dissipation 60 se présente sous la forme d'une plaque curviligne comprenant une ouverture 64 pour le passage du câble d'alimentation et des évidements 68 pour les yeux de l'utilisateur. L'ouverture 64 comprend également sur un bord périphérique une languette 66 percée pour le passage du câble d'alimentation électrique et de l'agencement de presse-étoupe 70. De préférence, chaque branche 54 s'étend symétriquement par rapport à un plan contenant la ligne méridienne M1.

De préférence, chaque branche 54 s'étend symétriquement par rapport à un plan contenant la ligne méridienne M1.

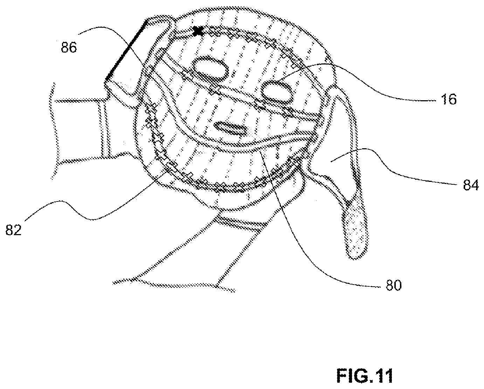

On a représenté sur les figures 10 et 11 le masque facial 10 selon un deuxième mode de réalisation de l'invention. Dans ce deuxième mode de réalisation, les éléments analogues à ceux du premier mode de réalisation portent des références identiques.

Dans le deuxième mode de réalisation, l'armature 12 est textile et comprend une pluralité de fibres optiques 80 en chaîne et/ou en trames tissées avec des fils de liage des fibres de l'armature 12, chaque fibre optique 80 comprenant un corps longitudinal pourvu selon sa direction longitudinale d'altérations invasives 82 ouvertes sur la face frontale de l'armature 12 et d'au moins une extrémité libre en regard de laquelle est agencée une source d'émission de lumière 86 pour transmettre et émettre la lumière au niveau des altérations 82 de la face frontale pour former la pluralité de points lumineux.

De préférence, la densité linéique et/ou la dimension des altérations 82 varie le long de chaque fibre optique 80 pour produire un éclairage surfacique variable depuis une face frontale de l'armature 12.

Par exemple, l'armature 12 est en toile et/ou en satin et dans lequel les fils de liage sont formés dans un matériau choisi parmi le groupe comprenant le polyamide le polyester le polyéthylène et le polypropylène.

On va maintenant décrire les principaux aspects du fonctionnement et de fabrication d'un appareil de traitement et dispositif de traitement selon l'invention.

En référence à la figure 1, lors de la mise sous tension du dispositif 100, l'utilisateur positionne le masque 10 en face de son visage de manière à ce que ces yeux coïncident avec les évidements 16 du masque 10. Le protocole de traitement peut alors démarrer.

En référence à la figure 11, l'utilisateur installe le masque 10 au moyen des attaches velcro 84 autour de sa tête. Le protocole de traitement peut être lancé.

Bien entendu, l'invention ne se limite pas aux modes de réalisation précédemment décrits. D'autres modes de réalisation à la portée de l'homme du métier peuvent aussi être envisagés sans sortir du cadre de l'invention définie par les revendications ci-après.

## Revendications

1. Masque facial (10) de traitement par photothérapie d'un visage d'un utilisateur, comprenant une armature (12) délimitant une face frontale orientée en direction d'un visage d'un utilisateur et une face dorsale opposée, l'armature (12) supportant une pluralité de points lumineux d'émission lumineuse (22) agencés dans le masque pour produire un éclairage énergétique du visage de l'utilisateur par la face frontale, le masque facial (10) définissant, en correspondance faciale, au moins une ligne remarquable dite méridienne (M1) du front au menton et une pluralité de lignes remarquables dites parallèles (P) s'étendant entre chaque côté du visage sensiblement transversalement à la ligne méridienne (M1), les points lumineux (22) étant disposés essentiellement le long des lignes parallèles (P) afin de produire une distribution spatiale de l'éclairement énergétique du visage présentant une densité de puissance lumineuse maximale le long de ces lignes (P), la densité de puissance lumineuse générée par les points lumineux (22) variant d'une ligne à l'autre ou le long d'une même ligne afin de définir des régions cibles de forte puissance lumineuse et des régions cibles de plus faible puissance lumineuse le long de la ligne ou d'une ligne à l'autre, **caractérisé en ce que** le taux de dosage énergétique lumineux variant le long d'une même ligne et/ou d'une ligne à l'autre permet d'ajuster le taux de dosage énergétique lumineux dans une ligne orbitale inférieure et une ligne orbitale supérieure définissant une région péri-orbitale de telle sorte que l'éclairement énergétique dans la région péri-orbitale est moins élevé que dans une région malaire ou maxillaire du visage.

2. Masque facial (10) selon la revendication précédente, dans lequel les points lumineux (22) présentent une distribution spatiale observable depuis la face frontale du masque (10) définissant un motif de réseau de lignes distinctes et discernables les unes des autres.

3. Masque facial (10) selon la revendication précédente, dans lequel, le visage pouvant être décomposé en une pluralité de zones susceptibles d'être marquées par les rides, le réseau définit une pluralité de lignes de points lumineux agencées pour traverser lesdites zones, la longueur de chaque ligne du réseau étant dimensionnée en fonction d'une étendue transversale de la zone traversée par la ligne.

4. Masque facial (10) selon la revendication 2 ou 3, dans lequel le réseau comprend au moins une ligne curviligne, par exemple pour suivre un contour péri-orbital ou péri-buccal du visage.

5. Masque facial (10) selon l'une quelconque des revendications précédentes, dans lequel la densité de puissance lumineuse maximale générée par les points lumineux (22) pour une ligne remarquable (P) a une valeur comprise entre 5 et 100 mW/cm², en particulier entre 20 et 70 mW/cm².

6. Masque facial (10) selon l'une quelconque des revendications précédentes, dans lequel la densité linéique des points lumineux (22) varie le long d'une ligne et/ou d'une ligne à l'autre.

7. Masque facial (10) selon l'une quelconque des revendications précédentes, définissant une ligne circonférentielle qui suit une forme générale ovale périphérique du visage le long de laquelle est agencée une pluralité de points lumineux.

8. Masque facial (10) selon l'une quelconque des revendications précédentes, dans lequel une parmi la pluralité des lignes parallèles est choisie parmi une ligne orbitale inférieure, une ligne orbitale supérieure, une ligne maxillaire inférieure, une ligne frontale, une ligne maxillaire supérieure, une ligne malaire centrale.

9. Masque facial (10) selon l'une quelconque des revendications précédentes, dans lequel chaque point lumineux (22) étant formé par une diode électroluminescente (24), l'armature (12) comprend un support (20) de raccordement électrique des diodes électroluminescentes (24) entre elles, ledit support (20) comprenant au moins un tronc principal (52) s'étendant sensiblement selon la ligne méridienne (M1) et au moins deux branches (54) s'étendant sensiblement selon au moins deux lignes parallèles distinctes (P).

10. Masque facial (10) selon la revendication précédente, dans lequel chaque branche (54) s'étend symétriquement par rapport à un plan contenant la ligne méridienne (M1).

11. Masque facial (10) selon la revendication 9 ou 10, comprenant une pluralité de branches primaires (54) reliées directement au tronc (52) et au moins une branche secondaire (56) reliée indirectement au tronc (52) par l'intermédiaire d'au moins une branche primaire (54).

12. Masque facial (10) selon l'une quelconque des revendications 11 à 12, dans lequel le tronc principal (52) comprend un bornier de raccordement (50) à une extrémité d'un câble d'alimentation électrique des sources lumineuses (22).

13. Masque facial (10) selon l'une quelconque des revendications 6 à 9, dans lequel l'armature (12) comprend deux parois (30, 40) en matière plastique conformée pour présenter une concavité orientée en direction du visage à traiter et entre lesquelles s'étend le support (20) portant les points lumineux (22).

14. Masque facial (10) selon la revendication précédente, dans lequel l'armature (12) comprenant une plaque de dissipation thermique (60) conformée pour venir épouser la concavité du corps et disposée entre les deux parois (30, 40), sur lequel est monté le support (12) du côté de la plaque (60) faisant face à la paroi intérieure (30).

15. Masque facial (10) selon l'une quelconque des revendications 1 à 12, dans lequel l'armature (12) est textile et comprend une pluralité de fibres optiques (80) en chaîne et/ou en trames tissées avec des fils de liage des fibres de l'armature (12), chaque fibre optique (80) comprenant un corps longitudinal pourvu selon sa direction longitudinale d'altérations invasives (82) ouvertes sur la face frontale de l'armature (12) et d'au moins une extrémité libre en regard de laquelle est agencée une source d'émission de lumière (86) pour transmettre et émettre la lumière au niveau des altérations (82) de la face frontale pour former la pluralité de points lumineux (22).

16. Masque facial (10) selon la revendication précédente, dans lequel la densité linéique et/ou la dimension des altérations (82) varie le long de la fibre optique (80) ou d'une fibre optique (80) à l'autre.

17. Masque facial (10) selon la revendication 15 ou 16, dans lequel l'armature (12) en toile et ou en satin et dans lequel les fils de liage sont formés dans un matériau choisi parmi le groupe comprenant le polyamide le polyester le polyéthylène et le polypropylène.

18. Masque facial (10) selon l'une quelconque des revendications précédentes, dans lequel les points lumineux (22) émettent dans un spectre rouge de longueur d'onde comprise entre 620 et 640 nm.

## Patentansprüche

1. Gesichtsmaske (10) zur Phototherapiebehandlung des Gesichts eines Benutzers, umfassend ein Gestell (12), das eine in Richtung des Gesichts eines Benutzers orientierte Vorderseite und eine gegenüberliegende Rückseite begrenzt, wobei das Gestell (12) eine Vielzahl von Lichtemissionspunkten (22) trägt, die in der Maske angeordnet sind, um eine energetische Beleuchtung des Gesichts des Benutzers durch die Vorderseite zu erzeugen, wobei die Gesichtsmaske (10) in fazialer Entsprechung mindestens eine sogenannte Meridian-Referenzlinie (M1) von der Stirn zum Kinn und eine Vielzahl von sogenannten parallelen Referenzlinien (P) definiert, die sich zwischen jeder Seite des Gesichts im Wesentlichen quer zur Meridianlinie (M1) erstrecken, wobei die Lichtpunkte (22) im Wesentlichen entlang der parallelen Linien (P) angeordnet sind, um eine räumliche Verteilung der Bestrahlungsstärke des Gesichts zu erzeugen, die eine maximale Lichtleistungsdichte entlang dieser Linien (P) aufweist, wobei die von den Lichtpunkten (22) erzeugte Lichtleistungsdichte von einer Linie zur anderen oder entlang derselben Linie variiert, um Zielbereiche mit hoher Lichtleistung und Zielbereiche mit geringerer Lichtleistung entlang der Linie oder von einer Linie zur anderen zu definieren, **dadurch gekennzeichnet, dass** die entlang derselben Linie und/oder von einer Linie zur anderen variierende energetische Lichtdosierung es ermöglicht, die energetische Lichtdosierung in einer unteren Orbitallinie und einer oberen Orbitallinie anzupassen, die einen periorbitalen Bereich definieren, dergestalt, dass die Bestrahlungsstärke im periorbitalen Bereich niedriger ist als in einem malaren oder maxillären Bereich des Gesichts.

2. Gesichtsmaske (10) nach dem vorangehenden Anspruch, in der die Lichtpunkte (22) eine von der Vorderseite der Maske (10) aus beobachtbare räumliche Verteilung aufweisen, die ein Netzmuster aus voneinander unterscheidbaren und erkennbaren Linien definiert.

3. Gesichtsmaske (10) nach dem vorangehenden Anspruch, in der das Gesicht in eine Vielzahl von Bereichen unterteilt werden kann, die anfällig für Faltenbildung sind, wobei das Netz eine Vielzahl von Linien von Lichtpunkten definiert, die so angeordnet sind, dass sie die besagten Bereiche durchqueren, wobei die Länge jeder Linie des Netzes in Abhängigkeit von einer Querausdehnung des von der Linie durchquerten Bereichs dimensioniert ist.

4. Gesichtsmaske (10) nach Anspruch 2 oder 3, in der das Netz mindestens eine gekrümmte Linie umfasst, beispielsweise um einer periorbitalen oder peribukkalen Kontur des Gesichts zu folgen.

5. Gesichtsmaske (10) nach einem der vorangehenden Ansprüche, in der die von den Lichtpunkten (22) für eine Referenzlinie (P) erzeugte maximale Lichtleistungsdichte einen Wert zwischen 5 und 100 mW/cm², insbesondere zwischen 20 und 70 mW/cm², aufweist.

6. Gesichtsmaske (10) nach einem der vorangehenden Ansprüche, in der die Lineardichte der Lichtpunkte (22) entlang einer Linie und/oder von einer Linie zur anderen variiert.

7. Gesichtsmaske (10) nach einem der vorangehenden Ansprüche, die eine Umfangslinie definiert, die einer allgemeinen ovalen Randform des Gesichts folgt, entlang derer eine Vielzahl von Lichtpunkten angeordnet ist.

8. Gesichtsmaske (10) nach einem der vorangehenden Ansprüche, in der eine aus der Vielzahl der parallelen Linien ausgewählt ist aus einer unteren Orbitallinie, einer oberen Orbitallinie, einer unteren Maxillarlinie, einer Frontallinie, einer oberen Maxillarlinie oder einer zentralen Malarlinie.

9. Gesichtsmaske (10) nach einem der vorangehenden Ansprüche, in der jeder Lichtpunkt (22) durch eine Leuchtdiode (24) gebildet wird, wobei das Gestell (12) einen Träger (20) zur elektrischen Verbindung der Leuchtdioden (24) untereinander umfasst, wobei der Träger (20) mindestens einen Hauptstamm (52) umfasst, der sich im Wesentlichen entlang der Meridianlinie (M1) erstreckt, und mindestens zwei Arme (54), die sich im Wesentlichen entlang mindestens zweier verschiedener paralleler Linien (P) erstrecken.

10. Gesichtsmaske (10) nach dem vorangehenden Anspruch, in der sich jeder Arm (54) symmetrisch zu einer Ebene erstreckt, welche die Meridianlinie (M1) enthält.

11. Gesichtsmaske (10) nach Anspruch 9 oder 10, umfassend eine Vielzahl von Primärarmen (54), die direkt mit dem Stamm (52) verbunden sind, und mindestens einen Sekundärarm (56), der indirekt über mindestens einen Primärarm (54) mit dem Stamm (52) verbunden ist.

12. Gesichtsmaske (10) nach einem der Ansprüche 11 bis 12 (sic), in der der Hauptstamm (52) eine Anschlussleiste (50) an einem Ende eines Stromversorgungskabels für die Lichtquellen (22) umfasst.

13. Gesichtsmaske (10) nach einem der Ansprüche 6 bis 9 (sic), in der das Gestell (12) zwei Kunststoffwände (30, 40) umfasst, die so geformt sind, dass sie eine in Richtung des zu behandelnden Gesichts orientierte Konkavität aufweisen, und zwischen denen sich der die Lichtpunkte (22) tragende Träger (20) erstreckt.

14. Gesichtsmaske (10) nach dem vorangehenden Anspruch, in der das Gestell (12) eine Wärmeableitplatte (60) umfasst, die so geformt ist, dass sie sich an die Konkavität des Körpers anschmiegt, und die zwischen den beiden Wänden (30, 40) angeordnet ist, wobei der Träger (12) auf der der Innenwand (30) zugewandten Seite der Platte (60) montiert ist.

15. Gesichtsmaske (10) nach einem der Ansprüche 1 bis 12, in der das Gestell (12) textil ist und eine Vielzahl von optischen Fasern (80) in Kette und/oder Schuss umfasst, die mit Bindefäden der Fasern des Gestells (12) verwebt sind, wobei jede optische Faser (80) einen Längskörper aufweist, der entlang seiner Längsrichtung mit invasiven Veränderungen (82) versehen ist, die zur Vorderseite des Gestells (12) hin offen sind, und mindestens ein freies Ende aufweist, gegenüber dem eine Lichtemissionsquelle (86) angeordnet ist, um das Licht im Bereich der Veränderungen (82) der Vorderseite zu übertragen und auszustrahlen, um die Vielzahl von Lichtpunkten (22) zu bilden.

16. Gesichtsmaske (10) nach dem vorangehenden Anspruch, in der die Lineardichte und/oder die Dimension der Veränderungen (82) entlang der optischen Faser (80) oder von einer optischen Faser (80) zur anderen variiert.

17. Gesichtsmaske (10) nach Anspruch 15 oder 16, in der das Gestell (12) aus Gewebe oder Satin besteht und in der die Bindefäden aus einem Material gebildet sind, das aus der Gruppe bestehend aus Polyamid, Polyester, Polyethylen und Polypropylen ausgewählt ist.

18. Gesichtsmaske (10) nach einem der vorangehenden Ansprüche, in der die Lichtpunkte (22) in einem roten Spektrum mit einer Wellenlänge zwischen 620 und 640 nm emittieren.

## Claims

1. A facial mask (10) for phototherapy treatment of a user's face, comprising a frame (12) delimiting a front face oriented towards a user's face and an opposite back face, the frame (12) supporting a plurality of light-emitting points (22) arranged in the mask to produce energetic lighting of the user's face through the front face, the facial mask (10) defining, in facial correspondence, at least one so-called meridian reference line (M1) from the forehead to the chin and a plurality of so-called parallel reference lines (P) extending between each side of the face substantially transversally to the meridian line (M1), the light points (22) being arranged essentially along the parallel lines (P) so as to produce a spatial distribution of the irradiance of the face having a maximum light power density along these lines (P), the light power density generated by the light points (22) varying from one line to another or along a same line so as to define target regions of high light power and target regions of lower light power along the line or from one line to another, **characterized in that** the light energy dosage rate varying along a same line and/or from one line to another allows adjusting the light energy dosage rate in a lower orbital line and an upper orbital line defining a peri-orbital region such that the irradiance in the peri-orbital region is lower than in a malar or maxillary region of the face.

2. The facial mask (10) according to the preceding claim, wherein the light points (22) have a spatial distribution observable from the front face of the mask (10) defining a network pattern of distinct and discernible lines from each other.

3. The facial mask (10) according to the preceding claim, wherein, the face being decomposable into a plurality of zones susceptible to being marked by wrinkles, the network defines a plurality of lines of light points arranged to cross said zones, the length of each line of the network being dimensioned according to a transverse extent of the zone crossed by the line.

4. The facial mask (10) according to claim 2 or 3, wherein the network comprises at least one curvilinear line, for example to follow a peri-orbital or peri-buccal contour of the face.

5. The facial mask (10) according to any one of the preceding claims, wherein the maximum light power density generated by the light points (22) for a reference line (P) has a value between 5 and 100 mW/cm², in particular between 20 and 70 mW/cm².

6. The facial mask (10) according to any one of the preceding claims, wherein the linear density of the light points (22) varies along a line and/or from one line to another.

7. The facial mask (10) according to any one of the preceding claims, defining a circumferential line that follows a general oval peripheral shape of the face along which a plurality of light points is arranged.

8. The facial mask (10) according to any one of the preceding claims, wherein one of the plurality of parallel lines is chosen from a lower orbital line, an upper orbital line, a lower maxillary line, a frontal line, an upper maxillary line, and a central malar line.

9. The facial mask (10) according to any one of the preceding claims, wherein each light point (22) being formed by a light-emitting diode (24), the frame (12) comprises a support (20) for the electrical connection of the light-emitting diodes (24) to each other, said support (20) comprising at least one main trunk (52) extending substantially along the meridian line (M1) and at least two branches (54) extending substantially along at least two distinct parallel lines (P).

10. The facial mask (10) according to the preceding claim, wherein each branch (54) extends symmetrically with respect to a plane containing the meridian line (M1).

11. The facial mask (10) according to claim 9 or 10, comprising a plurality of primary branches (54) directly connected to the trunk (52) and at least one secondary branch (56) indirectly connected to the trunk (52) via at least one primary branch (54).

12. The facial mask (10) according to any one of claims 11 to 12 (sic), wherein the main trunk (52) comprises a connection terminal block (50) at one end of an electrical power supply cable for the light sources (22).

13. The facial mask (10) according to any one of claims 6 to 9 (sic), wherein the frame (12) comprises two walls (30, 40) made of plastic material shaped to present a concavity oriented towards the face to be treated and between which the support (20) carrying the light points (22) extends.

14. The facial mask (10) according to the preceding claim, wherein the frame (12) comprises a heat dissipation plate (60) shaped to fit the concavity of the body and disposed between the two walls (30, 40), on which the support (12) is mounted on the side of the plate (60) facing the inner wall (30).

15. The facial mask (10) according to any one of claims 1 to 12, wherein the frame (12) is textile and comprises a plurality of optical fibers (80) in warp and/or woven weft with binding yarns of the fibers of the frame (12), each optical fiber (80) comprising a longitudinal body provided along its longitudinal direction with invasive alterations (82) open on the front face of the frame (12) and at least one free end opposite which a light emission source (86) is arranged to transmit and emit light at the level of the alterations (82) of the front face to form the plurality of light points (22).

16. The facial mask (10) according to the preceding claim, wherein the linear density and/or the dimension of the alterations (82) varies along the optical fiber (80) or from one optical fiber (80) to another.

17. The facial mask (10) according to claim 15 or 16, wherein the frame (12) is made of canvas and/or satin and wherein the binding yarns are formed of a material chosen from the group comprising polyamide, polyester, polyethylene, and polypropylene.

18. The facial mask (10) according to any one of the preceding claims, wherein the light points (22) emit in a red spectrum with a wavelength between 620 and 640 nm.
